# EUROPÄISCHE PATENTSCHRIFT

(11) **EP 0 338 427 B1**
(45) Veröffentlichungstag und Bekanntmachung des Hinweises auf die Patenterteilung: **18.08.1993**
(21) Anmeldenummer: 89106599.7
(22) Anmeldetag: 13.04.1989
(51) Int. Cl.: C07C 69/533, C07C 67/32

(54) **Verfahren zur Herstellung von olefinisch ungesättigten, endständigen Carbonsäureestern**
Process for the preparation of esters olefinic unsaturated carboxylic acids with a terminal ester group
Procédé de préparation d'esters d'acides carboxyliques insaturés oléfiniques à fonction ester terminale

(30) Priorität: 20.04.1988 DE 3813147
(43) Veröffentlichungstag der Anmeldung: 25.10.1989
(73) Patentinhaber: BASF Aktiengesellschaft, 67063 Ludwigshafen (DE)
(72) Erfinder: Merger, Franz, Dr., D-6710 Frankenthal (DE); Horler, Hans, Dr., D-6100 Darmstadt (DE); Hoelderich, Wolfgang, Dr., D-6710 Frankenthal (DE); Witzel, Tom, Dr., D-6700 Ludwigshafen (DE)

(56) Entgegenhaltungen:
- EP-A- 0 133 548

## Beschreibung

Die vorliegende Erfindung betrifft ein Verfahren zur Herstellung von olefinisch ungesättigten, endständigen Carbonsäureestern durch Retrocarbonylierung von α-substituierten α,ω-(n-Alkylendicarbonsäureestern).

Aus der EP-A-135 436 ist bekannt, daß Carbonsäuren und deren Ester mit einer endständigen Estergruppe an Katalysatoren, die neben Nickel mindestens eines der Metalle Zinn, Germanium oder Blei enthalten, bei 200 bis 400°C zu Alkenen führen, deren Kohlenstoffgerüst um ein C-Atom geringer ist als das der Ausgangsstoffe.

Nach US-A-4 102 938 erhält man durch Thermolyse von Pflanzenölen (Glyceride von n-Carbonsäuren) in Gegenwart von Übergangsmetall-dotierten Silicium-Aluminiumoxid-Katalysatoren bei 300 bis 700°C Kohlenwasserstoffgemische, die neben Olefinen gesättigte Kohlenwasserstoffe und auch Crackprodukte enthalten.

Aus der japanische Offenlegungsschrift Nr. 47904/1975 (Anmelde-Nr. 95 944/1973) ist die Retrocarbonylierung aliphatischer und alicyclischer Carbonsäureester mit Phosphor-Wolfram-Oxide auf Siliciumoxid bekannt. So entstehen aus Isobutyraten in der Gasphase bei ca. 250°C Propylen und Kohlenmonoxid sowie das dem gebundenen Alkohol entsprechenden Alken.

Nach der DE-A-27 26 106 lassen sich Monocarbonsäuren mit einer endständigen oder α-substituierten Carboxylgruppe oder deren Ester in der Gasphase bei 250 bis 800°C an Aluminium-Boroxid-Katalysatoren unter Bildung ungesättigter Kohlenwasserstoffe dehydrierend decarboxylieren, wobei partielle Gerüstumlagerungen beobachtet wurden.

Aus der US-A-3 530 198 ist bekannt, daß mit Hilfe eines Katalysatorkomplexes, bestehend aus einem Edelmetall und z.B. Diphenylphosphin, ein Gemisch aus einem endständigen und einem α-substituierten Carbonsäureester unter Retrocarbonylierung unterschiedslos in das Olefin überfüht werden kann.

Der Erfindung lag nun die Aufgabe zugrunde, aus Dicarbonsäureestern selektiv diejenige Carbonsäureestergruppe zu entfernen, die am höher substituierten C-Atom steht.

Diese Aufgabe wird gelöst in einem Verfahren zur Herstellung von olefinisch ungesättigten, endständigen Carbonsäureestern der allgemeinen Formel Ia und Ib

R¹-CH₂-CH=CH-(CH₂)ᵣ₋₁-COOR² (Ia)

R¹-CH=CH-(CH₂)ᵣ-COOR² (Ib)

und gegebenenfalls weiterer Doppelbindungsisomerer, in denen
- R¹: Wassserstoff oder einen organischen Rest,
- R²: C₁- bis C₈-Alkyl und
- r: 1 bis 20
bedeuten, durch Retrocarbonylierung, welches dadurch gekennzeichnet ist, daß man α-substituierte α,ω-(n-Alkylendicarbonsäureester) der allgemeinen Formel II 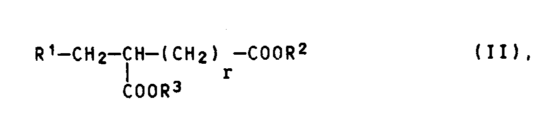
in der
- R¹: und R² sowie r die oben genannten Bedeutungen haben und
- R³: C₁- bis C₈-Alkyl
bedeutet, an einem aciden Heterogenkatalysator bei Temperaturen von 150 bis 800°C und Drücken von 0,01 bis 50 bar umsetzt.

Die olefinisch ungesättigten, endständigen Carbonsäureester sind nach folgender Methode erhältlich:
Die Umsetzung erfolgt durch Kontakt eines α-substituierten α,ω-(n-Alkylendicarbonsäureesters) II mit einem Katalysator nach folgender Reaktionsgleichung:

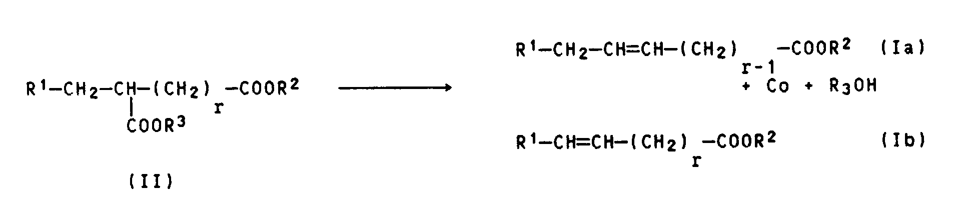

Die Alkencarbonsäureester Ia und Ib können unter den Reaktionsbedingungen weitere Doppelbindungsisomere bilden, sofern R¹ und r dies zulassen.

Die Reaktion kann diskontinuierlich oder vorzugsweise kontinuierlich, bevorzugt in der Gasphase bei 150 bis 800°C und 0,01 bis 50 bar durchgeführt werden.

Es kann jedoch auch eine Flüsigphasenreaktion bei Temperaturen von 50 bis 200°C und Drücken von 0,5 bis 5 bar durchgeführt werden.

Die bevorzugte Gasphasenreaktion kann beispielsweise bei Temperaturen von 150 bis 800°C, vorzugsweise bei 200 bis 600°C und Drücken von 0,1 bis 5 bar, besonders bevorzugt bei 280 bis 500°C und Drücken von 0,5 bis 2 bar durchgeführt werden. Bei der Umsetzung in der Gasphase hält man vorteilhaft eine Katalysatorbelastung von 0,01 bis 40, insbesondere von 0,05 bis 10 g Ausgangsstoff der Formel II je g Katalysator und Stunde ein (WHSV = g Einsatzgemisch/g Katalysator und Stunde). Die Gasphasenreaktion kann in einem Festbett oder in einem Wirbelbett ausgeführt werden.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Der Substituent R¹ in den Formeln Ia, Ib und II kann neben Wasserstoff für Alkylreste, z.B. mit 1 bis 10, insbesondere 1 bis 8 Kohlenstoffatomen wie Methyl, Ethyl, Propyl, iso-Propyl, Butyl, iso-Butyl, tert.-Butyl, Pentyl, iso-Pentyl, Hexyl, Heptyl oder Octyl; für Cycloalkylreste z.B. mit 4 bis 8, insbesondere 5 oder 6 Kohlenstoffatomen wie Cyclopentyl oder Cyclohexyl, für Aryl oder Aralkyl stehen. Arylreste sind z.B. Phenyl oder inerte Substituenten tragende Phenylreste wie C₁-C₄-alkyl, C₁-C₄-alkoxy-oder halogensubstituierte Phenylreste. Aralkylreste sind z.B. Benzylreste, wobei der Phenylkern die voranstehend genannten Substituenten tragen kann. Besonders bevorzugt sind Wasserstoff und Methyl.

Die Reste R² und R³ in den Formeln Ia, Ib und II stehen unabhängig voneinander für Alkylreste mit 1 bis 8, vorzugsweise 1 bis 4 Kohlenstoffatomen, wie Methyl, Ethyl, Propyl, iso-Propyl, Butyl, iso-Butyl und besonders bevorzugt für Methyl.

Der Index r in den Formeln Ia, Ib und II bedeutet 1 bis 20, bevorzugt 1 bis 8, besonders bevorzugt 1 bis 4.

Ausgangsstoffe der Formel II und in den Klammern die entsprechenden Endstoffe sind beispielsweise:

| | |
|---|---|
| Methylbernsteinsäuredimethylester | (→ n-Butensäuremethylester) |
| Ethylbernsteinsäuredimethylester | (→ n-Pentensäuremethylester) |
| n-Propylbernsteinsäuredimethylester | (→ n-Hexensäuremethylester) |
| n-Butylbernsteinsäuredimethylester | (→ n-Heptensäuremethylester) |
| n-Pentylbernsteinsäuredimethylester | (→ n-Octensäuremethylester) |
| 2-Methylglutarsäuredimethylester | (→ n-Pentensäuremethylester) |
| 2-Ethylglutarsäuredimethylester | (→ n-Hexensäuremethylester) |
| 2-(n-Propyl)glutarsäuredimethylester | (→ n-Heptensäuremethylester) |
| 2-(n-Butyl)glutarsäuredimethylester | (→ n-Octensäuremethylester) |
| 2-Methyladipinsäuredimethylester | (→ n-Hexensäuremethylester) |
| 2-Ethyladipinsäuredimethylester | (→ n-Heptensäuremethylester) |
| 2-(n-Propyl)adipinsäuredimethylester | (→ n-Octensäuremethylester) |
| 2-Methylpivalinsäuredimethylester | (→ n-Heptensäuremethylester) |
| 2-Ethylpivalinsäuredimethylester | (→ n-Octensäuremethylester) |
| Methylbernsteinsäurediethylester | (→ n-Butensäureethylester) |
| Ethylbernsteinsäurediethylester | (→ n-Pentensäureethylester) |
| n-Propylbernsteinsäurediethylester | (→ n-Hexensäureethylester) |
| n-Butylbernsteinsäurediethylester | (→ n-Heptensäureethylester) |
| n-Pentylbernsteinsäurediethylester | (→ n-Octensäureethylester) |
| 2-Methylglutarsäurediethylester | (→ n-Pentensäureethylester) |
| 2-Ethylglutarsäurediethylester | (→ n-Hexensäureethylester) |
| 2-(n-Propyl)glutarsäurediethylester | (→ n-Heptensäureethylester) |
| 2-(n-Pentyl)glutarsäurediethylester | (→ n-Octensäureethylester) |
| 2-Methyladipinsäurediethylester | (→ n-Hexensäureethylester) |
| 2-Ethyladipinsäurediethylester | (→ n-Heptensäureethylester) |
| 2-(n-Propyl)adipinsäurediethylester | (→ n-Octensäureethylester) |
| 2-Methylpivalinsäurediethylester | (→ n-Heptensäureethylester) |
| 2-Ethylpivalinsäurediethylester | (→ n-Octensäureethylester) |

Als acide Heterogenkatalysatoren verwendet man insbesondere saure Zeolithe, Phosphate oder sauer wirkende Oxide von Elementen der dritten und vierten Hauptgruppe sowie der zweiten bis sechsten Nebengruppe des periodischen Systems.

Besonders vorteilhaft werden zeolithische Katalysatoren eingesetzt.

Zeolithe sind kristalline Aluminiumsilikate, die eine hochgeordnete Struktur mit einem starren dreidimensionalen Netzwerk von SiO₄- und AlO₄-Tetraedern aufweisen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions ausgeglichen, ein Kationenaustausch ist möglich.

Für das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabile" Zeolithe des Faujasittyps, d.h. dealuminierte Zeolithe.

Besonders vorteilhaft sind Zeolithe mit Pentasilstruktur. Diese haben als Grundbaustein einen aus SiO₄-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignen sich die Alumino-, Boro- und Eisensilikatzeolithe mit Pentasilstruktur für das erfindungsgemäße Verfahren.

Der Aluminosilikatzeolithe werden z.B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Auch gehören hierzu die isotaktischen Zeolithe. Die erhaltenen Aluminosilikatzeolithe enthalten je nach Wahl der Einsatzstoffmengen ein SiO₂/Al₂O₃-Verhältnis von 10 bis 40.000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200°C unter autogenem Druck synthetisiert, indem man eine Borverbindung z.B. H₃BO₃, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise Fe₂(SO₄)₃ und einer Siliciumverbindung, vorzugsweise hochdispersem Siliciumdioxid, in wäßriger Aminlösung, insbesondere 1,6-Hexandiamin, mit oder ohne Alkali- oder Erdalkalizusatz bei 100 bis 220°C unter autogenem Druck.

Zu den verwendbaren siliciumreichen Zeolithen (SiO₂/Al₂O₃) gehören auch die sogenannten ZSM-Typen, Ferrierit, Nu-1 und Silicalite® (ein Molekularsieb, ein sog. Silica-Polymorph).

Die so hergestellten Alumino-, Boro- und Eisensilikatzeolithe können nach ihrer Isolierung, Trocknung bei 100 bis 160°C, vorzugsweise 105 bis 115°C und Calcinierung bei 450 bis 550°C, vorzugsweise 500°C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem SiO₂/Al₂O₃-Verhältnis von 25:75 bis 90:5, bevorzugt 75:25, Siliciumdioxid, bevorzugt hochdisperses SiO₂, Gemische aus hochdispersem SiO₂ und hochdispersem Al₂O₃, TiO₂, ZrO₂ sowie Ton. Nach der Verformung werden die Extrudate oder Preßlinge bei 110°C/16 h getrocknet und bei 500°C/16 h calciniert.

Man erhält auch vorteilhafte Katalysatoren, wenn der isolierte Alumino-bzw. Borosilikatzeolith direkt nach der Trocknung verformt wird und erst nach der Verformung einer Calcinierung unterworfen wird. Die hergestellten Alumino- ud Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen der zeolithischen Katalysatoren eventuell eine durch Koksabscheidung bedingte Desaktivierung eintritt, empfiehlt es sich, die Zeolithe durch Abtrennen der Koksablagerung mit Luft oder mit einem Luft/N₂-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Nebengruppe wie Al, Ga, Ge, Sn, Pb, Bi, Übergangsmetalle oder 4. bis 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Fr, Yb und U eingesetzt. Der Gehalt an diesen Metallen beträgt vorteilhaft von 0,1 bis 2,0 Gew.%.

Zweckmäßigerweise führt man die Dotierung so durch, daß man z.B. den verformten Zeolithen in einem Steigrohr vorlegt und bei 20 bis 100°C z.B. in eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Akaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cu(NO₃)₂ x 3 H₂O oder Ni(NO₃)₂ x 6 H₂O oder Ce(NO₃)₃ x 6 H₂O oder La(NO₃)₂ x 6 H₂O oder Cs₂CO₃ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150°C getrocknet und bei ca. 550°C calciniert. Dieser Tränkvorgang kann mehrmals hintereinander vorgenommen werden, um den gewünschten Metallgehalt einzustellen.

Auch ist es möglich, z.B. eine wäßrige Ni(CO₃)₂-Lösung oder ammoniakalische Pd(NO₃)₂-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcinierung bei ca. 500°C kann das so gewonnene zeolithische Material mit oder ohne Bindemittel zu Strängen, Pellets oder Wirbelgut weiterverarbeitet werden.

Ein Ionenaustausch des in der H-Form, Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige Ni(NO₃)₂-Lösung oder ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei ca. 150°C getrocknet und bei ca. 550°C calciniert. Bei manchen metalldotierten Zeolithen, z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 gew.%igen, insbesondere 12 bis 20 gew.%igen Salzsäure. Anschließend wird der so behandelte Zeolith mit Wasser gewaschen, getrocknet und bei 400 bis 500°C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 bis 160°C, getrocknet und bei Temperaturen von im allgemeinen 450 bis 600°C, calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 bis 90°C, vorzugsweise 60 bis 80°C, über einen Zeitraum von 0,5 bis 5, vorzugsweise mit 12 bis 20 gew.%iger Salzsäure, behandelt. Anschließend wird das zeolithische Material im allgemeinen ausgewaschen und zweckmäßig, z.B. bei Temperaturen von 100 bis 160°C, getrocknet und bei Temperaturen von im allgemeinen 450 bis 600°C calciniert. Einer HF-Behandlung kann sich auch eine HCl-Behandlung anschließen.

Nach einer anderen Arbeitsweise lassen sich Zeolithe durch Aufbringen von Phosphorverbindungen, wie Trimethoxiphosphat, Trimethoxiposphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger NaH₂PO₄-Lösung getränkt, bei 110°C getrocknet und bei 500°C calciniert.

Als acide Heterogenkatalysatoren können auch Schichtsilikate wie Montmorilonit und Bentonit verwendet werden.

Weitere Katalysatoren für das erfindungsgemäße Verfahren sind Phosphate, insbesondere Aluminiumphosphate, Siliciumaluminiumphosphate, Siliciumeisenaluminiumphosphate, Cerphosphate, Zirkonphosphate, Borphosphate, Eisenphosphate oder deren Gemische.

Als Aluminiumphosphat-Katalysatoren werden für das erfindungsgemäße Verfahren insbesondere unter hydrothermalen Bedingungen synthetisierte Aluminiumphosphate eingesetzt.

Die unter hydrothermalen Bedingungen hergestellten Aluminiumphosphate (APO) sind z.B. APO-5, APO-9, APO-11, APO-12, APO-14, APO-21, APO-25, APO-31 und APO-33. Synthesen dieser Verbindungen sind in der EP-A-132 708; US-A-4 310 440 und US-A-4 473 663 beschrieben.

Beispielsweise wird das AlPO₄-5 (APO-5) synthetisiert, indem man Orthophosphorsäure mit Pseudoboehmit (Catapal SB®) in Wasser homogen mischt; zu dieser Mischung Tetrapropylammoniumhydroxid gibt und danach bei ca. 150°C 20 bis 60 h unter autogenem Druck in einem Autoklaven umsetzt. Das abfiltrierte AlPO₄ wird getrocknet bei 100 bis 160°C und calciniert bei 450 bis 550°C.

AlPO₄-9 (APO-9) wird ebenfalls aus Orthophosphorsäure und Pseudoboehmit aber in wäßriger 1,4-Diazabicyclo-(2,2,2)octan-Lösung bei ca. 200°C unter autogenem Druck während 200 bis 400 h synthetisiert.

Die Synthese des AlPO₄-21 (APO-21) erfolgt aus Orthophosphorsäure und Pseudoboehmit in wäßriger Pyrrolidon-Lösung bei 150 bis 200°C unter autogenem Druck während 50 bis 200 h.

Die für das erfindungsgemäße Verfahren verwendbaren Siliciumaluminiumphosphate (SAPO) sind z.B. SAPO-5, SAPO-11, SAPO-31 und SAPO-34. Die Synthese dieser Verbindung wird z.B. in EP-A-103 117 oder US-A-4 440 871 beschrieben. Siliciumaluminiumphosphate werden hergestellt durch Kristallisation aus wäßriger Mischung bei 100 bis 250°C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von SiO₂ suspendiert in wäßriger Tetrapropylammoniumhydroxid-Lösung mit einer wäßrigen Suspension aus Pseudoboehmit und Orthophosphorsäure und anschließende Umsetzung bei 150 bis 200°C während 20 bis 200 h unter autogenem Druck in einem Rührautoklaven erhalten. Die Trocknung des abfiltrierten Pulvers bei 110 bis 160°C und die Calcination bei 450 bis 550°C.

Borphosphate für das erfindungsgemäße Verfahren lassen sich beispielsweise durch Mischung und Kneten von konzentrierter Borsäure und durch anschließende Trocknung und Calcination in Inertgas-, Luft- oder Dampfatmosphäre bei 250 bis 650°C, vorzugsweise 300 bis 500°C herstellen.

Als Phosphat-Katalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g Al(NO₃)₃ x H₂O in 700 ml Wasser über 2 h zugetropft. Dabei wird der pH-Wert durch gleichzeitige Zugabe von 25 %iger NH₃-Lösung bei pH 8 gehalten. Der entstandene Niederschlag wird 12 h nachgerührt, dann abgesaugt und ausgewaschen. Er wird bei 60°C/16 h getrocknet.

Ein für das erfindungsgemäße Verfahren eingesetztes Cerphosphat wird z.B. durch Fällung aus 52 g Ce(NO₃)₃ x 6 H₂O und 56 g NaH₂PO₄ x 2 H₂O erhalten. Nach der Filtration wird das Material zu Strängen veformt, bei 120°C getrocknet und bei 450°C calciniert.

Auf diese Phosphate können durch Imprägnierung (Tränken und Aufsprühen) oder in manchen Fällen auch durch Ionenaustausch Modifizierungskomponenten wie voran bei den Zeolithen beschrieben aufgebracht werden. Auch kann wie bei den Zeolithkatalysatoren eine Modifizierung mit Säure, z.B. Phosphorsäure erfolgen.

Ein Phosphorsäure-haltiger Katalysator kann beispielsweise durch Auftränken von H₃PO₄- oder NaH₂HPO₄- oder Na₂HPO₄-Lösung auf einen Träger wie SiO₂ und anschließende Trocknung bzw. Calcination erhalten werden. Phosphorsäure kann aber auch mit Kieselgel zusammen mit einem Sprühturm versprüht werden, danach schließt sich eine Trocknung und meist eine Calcination an. Phosphorsäure kann auch in einer Imprägniermühle auf das Trägermaterial aufgesprüht werden.

Weiterhin können für das erfindungsgemäße Verfahren vorteilhaft sauer wirkende Oxide, z.B. solche von Elementen der dritten und vierten Hauptgruppe sowie der zweiten bis sechsten Nebengruppe des periodischen Systems, insbesondere Oxide wie Siliciumdioxid in Form von Kieselgel, Kieselgur, Quarz, weiterhin Zinkoxid, Titandioxid, Zirkondioxid, Phosphoroxide, Vanadiumoxide, Nioboxide, Boroxide, Aluminiumoxide, Chromoxide, Molybdänoxide, Wolframoxide oder Bims oder Gemische dieser Oxide verwendet werden. Oxidgemische vorgenannter Oxide sind z.B. Aluminiumoxid wie γ-Al₂O₃ mit Boroxid, Siliciumdioxid, Wolframoxid oder Chromoxid. Die Oxide können durch Aufbringen von Modifizierungskomponenten wie voranstehend bei den Zeolithkatalysatoren beschrieben dotiert werden. Die Behandlung mit Säuren wie bei den Zeolithkatalysatoren beschrieben ist ebenfalls eine Möglichkeit der Modifizierung.

Die hier beschriebenen Katalysatoren können wahlweise als 2- bis 4 mm-Stränge oder als Tabletten mit 3 bis 5 mm Durchmesser oder als Splitt mit Teilchengrößen von 0,05, insbesondere 0,1 bis 0,5 mm oder als Wirbelkontakt eingesetzt werden. Das Wirbelgut läßt sich z.B. durch Zerkleinern und Aussieben von Strängen oder auch Sprühtrocknung herstellen.

### Beispiele

### Beispiel 1

### Herstellung von 2-, 3-, 4-Pentensäuremethylester aus 2-Methylglutarsäuredimethylester/Ethylbernsteinsäuredimethylester

Aus einem Verdampfer wurden bei Normaldruck pro Stunde je 50 g eines Gemisches aus 81 Gew.% 2-Methylglutarsäuredimethylester und 19 Gew.% Ethylbernsteinsäuredimethylester mit je 100 l Stickstoff in einen auf 500°C beheizten Reaktor (Quarzrohr-Innendurchmesser 20 mm) eingeleitet, der mit 50 ml Katalysator, bestehend aus 50 Gew.% γ-Al₂O₃ und 50 Gew.% B₂O₃, gepreßt als 2-mm-Stränge und einem Schüttgewicht von 0,50 kg je Liter, gefüllt war. Die entstandenen Reaktionsdämpfe kondensierte man. Nach 4-stündigem Betrieb wurden 184 g Reaktionsgemisch der folgenden Zusammensetzung (quant. GC-Analyse) erhalten:
74,1 Gew.% 2-Methylglutarsäuredimethylester,
19,9 Gew.% Ethylbernsteinsäuredimethylester,
0,8 Gew.% 4-Pentensäuremethylester,
1,7 Gew.% 3-Pentensäuremethylester,
0,4 Gew.% 2-Pentensäuremethylester.
3,1 Gew.% andere Produkte, die jedoch keine in α-Stellung verzweigten Monocarbonsäureester enthalten.

### Beispiel 2

### Herstellung von 2-, 3-, 4-Pentensäuremethylester aus 2-Methylglutarsäuredimethylester/Ethylbernsteinsäuredimethylester in Gegenwart eines Borosilikatzeolithen

### Herstellung des Katalysators:

Der Borosilikatzeolith des Pentasiltyps wurde in einer hydrothermalen Synthese aus 640 g hochdispersen SiO₂, 122 g H₃BO₃, 8000 g einer 50 %igen wäßrigen 1,6-Hexandiamin-Lösung bei 170°C unter autogenem Druck in einem Rührautoklaven hergestellt. Nach Abfiltrieren und Auswaschen wurde das kristalline Reaktionsprodukt 24 Stunden bei 100°C getrocknet und 24 Stunden bei 500°C calciniert. Dieser Borosilikatzeolith enthielt 94,2 Gew.% SiO₂ und 2,3 Gew.% B₂O₃.

Mit diesem Material wurden durch Verformen mit einem Verformungshilfsmittel 2-mm-Stränge hergestellt, die bei 110°C 16 Stunden getrocknet und bei 500°C 24 Stunden calciniert werden.

### Umsetzung:

50 g eines Gemisches aus 78 Gew.% 2-Methylglutarsäuredimethylester und 22 Gew.% Ethylbernsteinsäuredimethylester wurden pro Stunde bei Atmosphärendruck verdampft und bei 350°C über den Borosilikatzeolithen (Schüttdichte 0,50 kg je Liter Katalysator; Rohrreaktorinnendurchmesser 20 mm) geleitet. Bei einer Katalysatorbelastung von 1 kg Estergemisch pro Liter Katalysator und Stunde sowie einer Inertgasbelastung von 1000 l Stickstoff pro Liter Katalysator und Stunde fielen nach 4 Stunden durch Kondensation 196g eines Produktgemisches der folgenden Zusammensetzung (quant. GC-Analyse) an:
74,7 Gew.% 2-Methylglutarsäuredimethylester,
21,2 Gew.% Ethylbernsteinsäuredimethylester,
1,2 Gew.% 4-Pentensäuremethylester,
1,9 Gew.% 3-Pentensäuremethylester,
0,2 Gew.% 2-Pentensäuremethylester.
0,8 Gew.% andere Produkte, die jedoch keine in α-Stellung verzweigte Monocarbonsäureester enthalten.

Dies entspricht einer Gesamt-Pentensäuremethylester-Selektivität von 82 % bei einem 2-Methylglutarsäure-/Ethylbernsteinsäuredimethylesterumsatz von 6 %.

### Beispiel 3

### Herstellung von 2-, 3-, 4-Pentensäuremethylester aus 2-Methylglutarsäuredimethylester/Ethylbernsteinsäuredimethylester in Gegenwart eines Borosilikatzeolithen

In einem Quarzrohr verdampfte man bei Atmosphärendruck pro Stunde 50 g eines Gemisches aus 78 Gew.% 2-Methylglutarsäuredimethylester und 22 Gew.% Ethylbernsteinsäuredimethylester und leitete die Eduktdampfe gemeinsam mit 50 Liter Stickstoff bei 400°C über 50 ml des analog Beispiel 2 hergestellten Borosilikatzeolithen (Schüttdichte 0,50 g pro Liter; Reaktorinnendurchmesser 20 mm); die Reaktionsdämpfe wurden kondensiert. Nach 4 Stunden erhielt man 186 g Reaktionsgemisch das sich folgendermaßen zusammensetzte:
60,7 Gew.% 2-Methylglutarsäuredimethylester,
21,0 Gew.% Ethylbernsteinsäuredimethylester,
3,6 Gew.% 4-Pentensäuremethylester,
6,2 Gew.% 3-Pentensäuremethylester sowie
2,6 Gew.% 2-Pentensäuremethylester.
6,1 Gew.% andere Produkte, die jedoch keine in α-Stellung verzweigte Monocarbonsäureester enthalten.

Dies entspricht einer Gesamt-Pentensäuremethylester-Selektivität von 73 % bei einem 2-Methylglutarsäure-/Ethylbernsteinsäuredimethylesterumsatz von 24 %.

### Beispiel 4

### Herstellung von 2-, 3-, 4-Pentensäuremethylester aus 2-Methylglutarsäuredimethylester/Ethylbernsteinsäuredimethylester in Gegenwart eines cäsiumhaltigen Borosilikatzeolithen

### Herstellung des Katalysators

Stränge des Borosilikatzeolithen (analog Beispiel 2 hergestellt) werden mit einer wäßrigen CsCO₃-Lösung imprägniert, danach bei 130°C getrocknet und bei 540°C/2 h calciniert. Der Cs-Gehalt betrug 0,6 Gew.%.

### Umsetzung:

Ein Gemisch aus 81 Gew.% 2-Methylglutarsäuredimethylester und 19 Gew.% Ethylbernsteinsäuredimethylester wurden bei 300°C unter Atmosphärendruck verdmapft und bei 300°C über 50 ml des cäsiumdotierten Borosilikatzeolithen (Schüttdichte 0,52 kg pro Liter, Reaktorinnendurchmesser 20 mm) geleitet. Bei einer Katalysatorbelastung von 1 kg Estergemisch pro Liter Kontakt und Stunde erhielt man durch Kondensation nach 4 Stunden 190 g eines Produktgemisches das sich laut quant. GC-Analyse folgendermaßen zusammensetzte:
74,1 Gew.% 2-Methylglutarsäuredimethylester,
17,4 Gew.% Ethylbernsteinsäuredimethylester,
2,0 Gew.% 4-Pentensäuremethylester,
4,1 Gew.% 3-Pentensäuremethylester und
2,0 Gew.% 2-Pentensäuremethylester.
0,4 Gew.% andere Produkte, die jedoch keine in α-Stellung verzweigte Monocarbonsäureester enthalten.

Dies entspricht einer Gesamt-Pentensäuremethylester-Selektivität von 89 % bei einem 2-Methylglutarsäure-/Ethylbernsteinsäuredimethylester-Umsatz von 13 %.

### Beispiel 5

### Herstellung von 2-, 3-, 4-Pentensäuremethylester aus 2-Methylglutarsäuredimethylester/Ethylbernsteinsäuredimethylester in Gegenwart eines Aluminiumsilikatzeolithen

### Herstellung des Katalysators:

Der Aluminosilikatzeolith mit Pentasil-Struktur wird unter hydrothermalen Bedingungen bei autogenem Druck und 150°C aus 65 g hochdispersem SiO₂, 20,3 g Al₂(SO₄)₃ x 18 H₂O in 1 kg 50 %iger wäßriger 1,6-Hexandiamin-Lösung in einem Rührautoklaven hergestellt. Nach Abfiltrieren und Auswaschen wird das kristalline Reaktionsprodukt 24 Stunden bei 110°C getrocknet und 24 Stunden bei 500°C calciniert. Dieser Aluminosilikatzeolith enthält 91,6 Gew.% SiO₂ und 4,6 Gew.% Al₂O₃. Der Katalysator wird mit einem Verformungshilfsmittel zu 2-mm-Strängen verformt, anschließend 16 Stunden bei 110°C getrocknet und 24 Stunden bei 500°C calciniert.

### Umsetzung:

Bei 300°C verdampfte man pro Stunde 50 g eines Estergemisches aus 78 Gew.% 2-Methylglutarsäuredimethylester und 22 Gew.% 2-Ethylbernsteinsäuredimethylester, leitete das gasförmige Estergemisch zusammen mit 50 Liter Stickstoff bei 300°C über 50 ml des Aluminosilikatzeolithen (Schüttdichte 0,50 kg pro Liter, Reaktorinnendurchmesser 20 mm) und kondensierte die Reaktionsdämpfe. Nach 4 Stunden Reaktionszeit wurden 196 g Reaktionsgemisch der folgenden Zusammensetzung erhalten:
62,9 Gew.% 2-Methylglutarsäuredimethylester,
20,8 Gew.% Ethylbernsteinsäuredimethylester,
1,3 Gew.% 4-Pentensäuremethylester,
5,7 Gew.% 3-Pentensäuremethylester und
1,8 Gew.% 2-Pentensäuremethylester.
7,5 Gew.% andere Produkte, die jedoch keine in α-Stellung verzweigte Monocarbonsäureester enthalten.

Dies entspricht einem 2-Methylglutarsäure-/Ethylbernsteinsäuredimethylesterumsatz von 18 % bei einer Gesamt-Pentensäuremethylester-Selektivität von 73 %.

## Patentansprüche

1. Verfahren zur Herstellung von olefinisch ungesättigten, endständigen Carbonsäureestern der allgemeinen Formel Ia und Ib
R¹-CH₂-CH=CH-(CH₂)ᵣ₋₁-COOR² (Ia)
R¹-CH=CH-(CH₂)ᵣ-COOR² (Ib)
und gegebenenfalls weiterer Doppelbindungsisomerer, in denen
R¹ Wassserstoff oder einen organischen Rest,
R² C₁- bis C₈-Alkyl und
r 1 bis 20
bedeuten, dadurch gekennzeichnet, daß man α-substituierte α,ω-(n-Alkylendicarbonsäureester) der allgemeinen Formel II 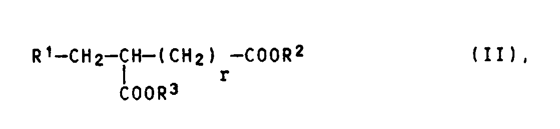 in der
R¹ und R² sowie r die oben genannten Bedeutungen haben und
R³ C₁- bis C₈-Alkyl
bedeutet, an einem aciden Heterogenkatalysator bei Temperaturen von 150 bis 800°C und Drücken von 0,01 bis 50 bar umsetzt.

2. Verfahren nach Anspruch 1, dadurch gekennzeichnet, daß die organischen Reste R¹ Alkyl, Cycloalkyl, Aryl oder Aralkyl bedeuten.

3. Verfahren nach den Ansprüchen 1 oder 2, dadurch gekennzeichnet, daß man als acide Heterogenkatalysatoren Oxide der dritten oder vierten Hauptgruppe und/oder der zweiten bis sechsten Nebengruppe des Periodensystems, Zeolithe oder Phosphate verwendet.

4. Verfahren nach Anspruch 1 bis 3, dadurch gekennzeichnet, daß man als Katalysatoren Pentasilzeolithe verwendet.

5. Verfahren nach Anspsruch 1 bis 4, dadurch gekennzeichnet, daß man als Katalysatoren Aluminium-, Eisen- und/oder Borsilikat verwendet.

6. Verfahren nach Anspruch 1 bis 5, dadurch gekennzeichnet, daß man als Katalysatoren Zeolithe verwendet, die durch Alkalimetalle und/oder Übergangsmetalle und/oder seltene Erdenmetalle dotiert oder mit einer anorganischen Säure behandelt sind.

7. Verfahren nach den Ansprüchen 1 bis 3, dadurch gekennzeichnet, daß man als acide Heterogenkatalysatoren Aluminiumphosphate, Siliciumaluminiumphosphate oder Borphosphate verwendet.

8. Verfahren nach den Ansprüchen 1 bis 3, dadurch gekennzeichnet, daß man als acide Heterogenkatalysatoren Aluminiumoxid, Siliciumoxid und/oder Boroxid verwendet.

9. Verfahren nach den Ansprüchen 1 bis 6, dadurch gekennzeichnet, daß man die Umsetzung in der Gasphase bei 150 bis 800°C durchführt.

## Claims

1. A process for the preparation of olefinically unsaturated carboxylic esters having a terminal ester group and of the formulae Ia and Ib
R¹-CH₂-CH=CH-(CH₂)ᵣ₋₁-COOR² (Ia)
R¹-CH=CH-(CH₂)ᵣ-COOR² (Ib)
and, if desired, further double bond isomers, where R¹ is hydrogen or an organic radical, R² is C₁-C₈-alkyl and r is from 1 to 20, which comprises converting an α-substituted α,ω-(n-alkylenedicarboxylic ester) of the formula II 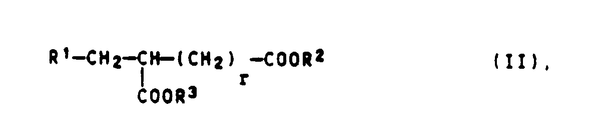 where R¹ and R² and r have the abovementioned meanings and R³ is C₁-C₈-alkyl, over an acidic heterogeneous catalyst at from 150 to 800°C and under from 0.01 to 50 bar.

2. A process as claimed in claim 1, wherein the organic radical R¹ is alkyl, cycloalkyl, aryl or aralkyl.

3. A process as claimed in claim 1 or 2, wherein the acidic heterogeneous catalysts used are oxides of main groups three or four and/or of subgroups two to six of the Periodic Table, zeolites or phosphates.

4. A process as claimed in any of claims 1 to 3, wherein the catalyst used is a pentasil zeolite.

5. A process as claimed in any of claims 1 to 4, wherein the catalysts used are aluminum silicate, iron silicate and/or boron silicate.

6. A process as claimed in any of claims 1 to 5, wherein the catalyst used is a zeolite which has been doped with alkali metals and/or transition metals and/or rare earth metals or treated with an inorganic acid.

7. A process as claimed in any of claims 1 to 3, wherein the acidic heterogeneous catalyst used is an aluminum phosphate, a silicon aluminum phosphate or a boron phosphate.

8. A process as claimed in any of claims 1 to 3, wherein the acidic heterogeneous catalysts used are alumina, silica and/or boron oxide.

9. A process as claimed in any of claims 1 to 6, wherein the reaction is carried out in the gas phase at from 150 to 800°C.

## Revendications

1. Procédé de préparation d'esters d'acides carboxyliques à insaturation oléfinique à fonction ester terminale de formules générales Ia et Ib
R¹-CH₂-CH=CH-(CH₂)ᵣ₋₁-COOR² (Ia)
R¹-CH=CH-(CH₂)ᵣ-COOR² (Ib)
et éventuellement d'autres isomères vis-à-vis de la double liaison, où
R¹ représente un atome d'hydrogène ou un reste organique,
R² représente un reste alkyle en C₁ à C₈ et
r est un nombre de 1 à 20,
caractérisé en ce qu'on fait réagir des esters d'acides α,ω-n-alkylènedicarboxyliques α-substitués de formule générale II 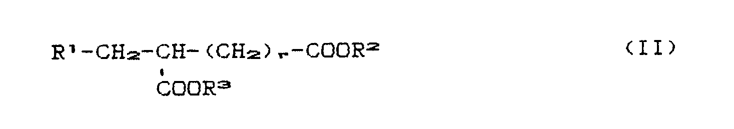 dans laquelle
R¹ et R² ainsi que r ont les significations données ci-dessus et
R³ représente un reste alkyle en C₁ à C₈,
en présence d'un agent de catalyse hétérogène acide, à des températures de 150 à 800°C et sous des pressions de 0,01 à 50 bar.

2. Procédé selon la revendication 1, caractérisé en ce que les restes organiques R¹ sont des restes alkyle, cycloalkyle, aryle ou aralkyle.

3. Procédé selon la revendication 1 ou 2, caractérisé en ce qu'on utilise, comme agents de catalyse hétérogène acides, des oxydes du groupe IIIA ou IVA et/ou des groupes IIB à VIB du système périodique, des zéolithes ou des phosphates.

4. Procédé selon l'une quelconque des revendications 1 à 3, caractérisé en ce qu'on utilise, comme catalyseurs, des zéolithes du type pentasil.

5. Procédé selon l'une quelconque des revendications 1 à 4, caractérisé en ce qu'on utilise, comme catalyseurs, un aluminosilicate, un ferrosilicate et/ou un borosilicate.

6. Procédé selon l'une quelconque des revendications 1 à 5, caractérisé en ce qu'on utilise, comme catalyseurs, des zéolithes qui ont été dopées avec des métaux alcalins et/ou des métaux de transition et/ou des métaux des terres rares ou ont été traitées par un acide minéral.

7. Procédé selon l'une quelconque des revendications 1 à 3, caractérisé en ce qu'on utilise, comme agents de catalyse hétérogène acides, des phosphates d'aluminium, des phosphates de silicium et d'aluminium ou des phosphates de bore.

8. Procédé selon l'une quelconque des revendications 1 à 3, caractérisé en ce qu'on utilise, comme agents de catalyse hétérogène acides, de l'oxyde d'aluminium, de l'oxyde de silicium et/ou de l'oxyde de bore.

9. Procédé selon l'une quelconque des revendications 1 à 6, caractérisé en ce qu'on conduit la réaction en phase gazeuse à une température de 150 à 800°C.
